# EUROPÄISCHE PATENTANMELDUNG

(11) **EP 2 997 942 A1**
(43) Veröffentlichungstag der Anmeldung: **23.03.2016**
(21) Anmeldenummer: 15002638.3
(22) Anmeldetag: 09.09.2015
(51) Int. Cl.: A61F 13/00, A61F 13/06, A61F 13/08, A61F 13/10, A61F 13/14, B22D 19/14, B29C 44/56, D04B 21/20

(54) **VERFAHREN ZUR HERSTELLUNG VON WUNDAUFLAGEN**

(30) Priorität: 15.09.2014 DE 102014013334
(71) Anmelder: RC-Cuijpers GmbH, 47574 Goch (DE)
(72) Erfinder: CUIJPERS, Rudolf, 5853 EE Siebengewald (NL)
(74) Vertreter: Döpp, Ludger

(57) **Zusammenfassung**

Die Erfindung betrifft ein Verfahren zur Herstellung elastisch verformter Wundauflagen indem biokompatible dehnbare Fäden oder Fasern dergestalt miteinander in Wirkverbindung gebracht werden, dass ein etwas flächiges Vorprodukt entsteht, dieses Vorprodukt in Abhängigkeit der zum Einsatz gelangenden Fäden oder Fasern bedarfsweise mit einer Paste oder einer Emulsion zumindest einseitig beschichtet wird und das Vorprodukt zur Erlangung der gewünschten geometrischen Kontur anschließend einem Verformungsprozess unterzogen wird.

## Beschreibung

Die Erfindung betrifft ein Verfahren zur Herstellung elastisch verformter Wundauflagen.

Allgemein bekannt ist, Netze und Tülle, insbesondere bei Verbrennungen, Abschürfungen der Haut, offenen Wunden oder dergleichen, als Wundauflagen einzusetzen. Netze/Tülle sind offene textile Flächengebilde, die sich durch hohe Flexibilität, einstellbare offene Maschenstruktur sowie eine gleich groß bleibende Perforierung auszeichnen. Diese Netze/Tülle werden als nicht elastische Wundauflagen eingesetzt.

Bei diesen Systemen werden nicht elastische Fäden eingesetzt. Somit ist eine Verformung der Netze/Tülle in der benötigten Tiefe nicht möglich. Da die Netze/Tülle keine Elastizität und Hysterese aufweisen, können diese Wundauflagen nur sehr eingeschränkt Bewegungsabläufen der Patienten folgen.

Durch ihre Art kommt es an den meisten Körperstellen zur Faltenbildung beim Anlegen. Darüber hinaus können diese Wundauflagen leicht verrutschen.

Der EP 2 159 255 A1 ist ein Verfahren zur Herstellung von geformten Polyurethanschaumwundauflagen zu entnehmen, wobei eine Schaumschicht verformt wird, welche einen Polyurethanschaum umfasst, der erhalten wird, indem eine Zusammensetzung, umfassend eine wässrige, anionisch hydrophilierte Polyurethandispersion aufgeschäumt und getrocknet wird, wobei das Verfahren bei einer Temperatur > 100° C bis < 200° C sowie bei einem Druck > 50 bar bis < 150 bar stattfindet und wobei weiterhin während des Verformens der Schaum auf > 25% bis < 100% seines ursprünglichen Volumens komprimiert wird.

In der DE 603 14 405 T2 werden ein chirurgischer Faden und ein chirurgisches Implantat mit diesem Faden beschrieben. Der Faden beinhaltet einen Kern, der aus mindestens einem resorbierbaren Material hergestellt und eine Ummantelung, die aus mindestens einem nicht resorbierbarem Material und/oder langsam resorbierbarem Material hergestellt ist, das langsamer resorbierbar als das resorbierbare Material des Kerns ist, wobei die Ummantelung Fäden umfasst. Die Fäden der Ummantelung sind in dem chirurgischen Faden als ein Einfachumwindezwirn oder Spinnwindezwirn angeordnet. Vor der Resorption des Kerns sind die Fäden durch den Kern gegen Zugkräfte formbeständig, wobei nach Resorption des Kerns die Formbeständigkeit fehlt, so dass sich die Ummantelung, wenn sie einer Zugkraft ausgesetzt wird, von ihrer nichtlinearen Anordnung in eine ungefähr lineare Anordnung bewegen kann. Der chirurgische Faden kann unter Verwendung einer Häkelgalontechnik hergestellt werden.

Der Erfindung liegt die Aufgabe zugrunde, ein Verfahren zur Herstellung elastisch verformter Wundauflagen bereitzustellen, wobei unter Einsatz geeigneter Fäden/Fasern eine hohe Elastizität der zu fertigenden Wundauflage erreicht werden kann.

Darüber hinaus liegt der Erfindung die Aufgabe zugrunde, eine nach diesem Verfahren hergestellte biokompatible Wundauflage vorzustellen, die an beliebige Körperstellen angepasst werden kann.

Diese Aufgabe wird gelöst durch ein Verfahren zur Herstellung elastisch verformter Wundauflagen, indem biokompatible dehnbare Fäden oder Fasern dergestalt miteinander in Wirkverbindung gebracht werden, dass ein etwa flächiges Vorprodukt entsteht, dieses Vorprodukt in Abhängigkeit der zum Einsatz gelangenden Fäden oder Fasern bedarfsweise mit einer Paste oder einer Emulsion zumindest einseitig beschichtet wird und das Vorprodukt zur Erzeugung der gewünschten geometrischen Kontur abschließend einem Verformungsprozess unterzogen wird.

Vorteilhafte Weiterbildungen des erfindungsgemäßen Verfahrens sind den zugehörigen verfahrensgemäßen Unteransprüchen zu entnehmen.

Die Aufgabe wird ebenfalls gelöst durch eine Wundauflage, hergestellt nach dem erfindungsgemäßen Verfahren. Die ebenfalls erfindungsgemäße Wundauflage ist bevorzugt einsetzbar zur Abdeckung von äußeren Verletzungen, insbesondere im Bereich der Brust, des Knies, der Ferse oder des Ellenbogens.

Unter dem Begriff "Biokompatibilität" versteht der Fachmann Materialien oder Werkstoffe, die keinen negativen Einfluss auf Lebewesen haben. Eine wesentliche Richtlinie wird hierbei durch die DIN-ISO 10993 gebildet.

Unter dem Begriff "Paste" versteht der Fachmann nicht mehr fließfähige Feststoff-Flüssigkeitsgemische mit einem großen Anteil an Festkörpern

Unter dem Begriff "Emulsion" versteht der Fachmann fein verteilte Gemische aus üblicherweise mischbaren Flüssigkeiten (ohne sichtbare Entmischung). Beispiele sind im Bereich kosmetischer Produkte angesiedelt.

An biokompatiblen Fäden oder Fasern kommen bevorzugt solche auf Basis von Polyester, Polyamid oder Polypropylen zum Einsatz. Des Weiteren denkbar sind Polyethylen- oder Seidenfäden Diese Fäden oder Fasern werden bevorzugt nicht verstreckt, haben somit ein gutes Dehnverhalten. Die Fäden oder Fasern können jedoch vor ihrer weiteren Verarbeitung geringfügig einer Verstreckung unterzogen werden, um damit die gewünschte Dehnung der Fäden oder Fasern zu modifizieren. Zum Einsatz gelangen hierbei handelsübliche Verstreckungseinrichtungen, die bedarfsweise mehrere Steckzonen enthalten können.

Alternativ besteht ebenfalls die Möglichkeit, Fäden oder Fasern auf Basis von Silikon einzusetzen. Alternativ zu Silikon können auch Polyurethanfäden eingesetzt werden. Derartige Fäden sind jedoch nicht weiterverarbeitbar, so dass selbige mit einer Ummantelung, bevorzugt auf Basis von Polyester, Polyamid oder Polypropylen versehen werden. Des Weiteren denkbar sind Ummantelungen auf Basis von Polyethylen bzw. Seide.

In Analogie zum Stand der Technik werden die Fäden oder Fasern dergestalt miteinander in Wirkverbindung gebracht, dass flächige Gewebe-, Gestricke-, Gewirke,- oder Non-woven-Vorprodukte gebildet werden. Wirk- und Strickwaren gehören beide zu den Maschenwaren, bei denen eine Fadenschlinge in eine andere geschlungen wird. Der Vorteil von gewirkten Textilien liegt in ihrer großen Elastizität.

Die meisten Gewebe werden durch einlagige Gewebe gebildet, beinhaltend jeweils nur ein Kettfaden- und ein Schussfadensystem.

Der Begriff "non-woven" beinhaltet u.a. Vliesstoffe, die größtenteils durch flexible textile Flächengebilde dargestellt sind.

Um die gewünschte hohe Elastizität der Wundauflage erreichen zu können wird vorgeschlagen, die vorteilhafterweise nicht oder nur geringfügig verstreckten Fäden oder Fasern auf Basis von Polyester, Polyamid oder Polypropylen mit einer Dehnung von mindestens 60% und einem Titer von 30 - 300 dtex, bevorzugt 50 - 80 dtex, zu versehen.

Die umwundenen Silikonfäden sollen hierbei eine Dehnung von 120 - 400% aufweisen.

Die Fäden und Fasern auf Basis von Polyester, Polyamid oder Polypropylen werden mit der angesprochenen Beschichtung versehen, wobei die umwundenen Silikonfäden, respektive Polyurethanfäden, nur bedarfsweise beschichtet werden müssen.

Zur Beschichtung des Vorprodukts können unterschiedlich viskose Materialien eingesetzt werden. Bevorzugt wird hierbei ein biokompatibles Silikon, insbesondere ein Zweikomponentensilikon, eingesetzt.

Alternativ besteht auch die Möglichkeit die Vorprodukte mit Vaseline oder Peru-Balsam zu beschichten. Ebenfalls denkbar sind hautverträgliche Öle bzw. Gele.

Entscheidende Parameter bei der Herstellung elastisch verformter Wundauflagen sind somit
- die Elastizität des Textil-Vorproduktes,
- die bedarfsweise vorzunehmende Beschichtung des Vorprodukts,
- die Verformbarkeit des Vorprodukts.

Die Beschichtung der Vorprodukte kann bspw. durch Rakeln, Sprühen oder Foulardieren erfolgen. Während beim Rakeln oder Sprühen einseitige Beschichtungen der Vorprodukte möglich sind, wird beim Foulardieren das gesamte Vorprodukt getränkt.

Die bisher auf Basis von Netzen oder Tüllen zum Einsatz gelangenden Wundauflagen neigen an vielen Körperstellen zur Faltenbildung und können somit leicht verrutschen. Die durch das erfindungsgemäße Verfahren hergestellten Wundauflagen sind spezifisch für die jeweiligen Körperstellen angepasst und liegen damit passgenau auf der Haut, so dass das Verrutschen minimiert und der Tragekomfort wesentlich erhöht ist.

Die hohe Elastizität der erfindungsgemäßen Wundauflagen erlaubt eine große Bewegungsfreiheit der Patienten. Die nach dem erfindungsgemäßen Verfahren hergestellten Wundauflagen finden ihren wesentlichen Einsatz bei Verbrennungen der weiblichen Brust (z.B. nach Bestrahlungen), Abschürfungen der Haut, insbesondere an Ellenbogen, Knien, Oberschenkeln, Handgelenken und Verletzungen der Ferse.

Die nach dem erfindungsgemäßen Verfahren hergestellte Wundauflage kann hierbei nach Bedarf mit oder ohne Adhäsion/Selbstklebewirkung hergestellt werden.

Der Erfindungsgegenstand ist anhand eines Ausführungsbeispiels in der Zeichnung dargestellt und wie folgt beschrieben. Es zeigen:
- Figur 1: Prinzipskizze eines Vorprodukts für eine erfindungsgemäße Wundauflage,
- Figur 2 - 3: unterschiedliche Beschichtungsverfahren zur Erzeugung erfindungsgemäßer Wundauflagen,
- Figur 4 - 5: Prinzipskizzen von Verformungsprozessen zur Erzeugung erfindungsgemäßer Wundauflagen,
- Figur 6: fertig geformte Wundauflage

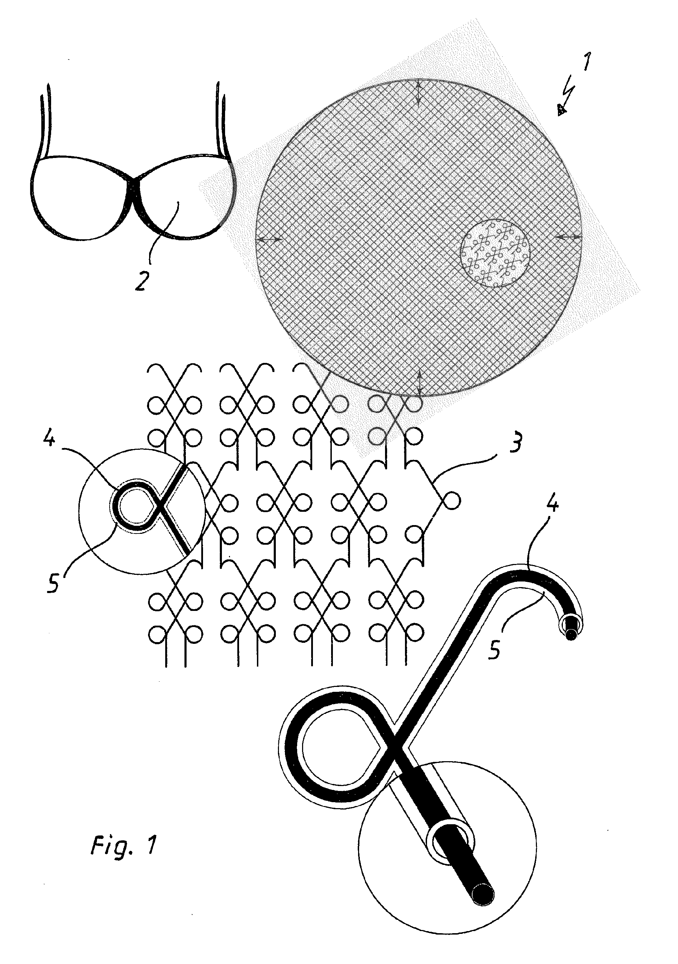

Figur 1 zeigt als Prinzipskizze das Vorprodukt 1 einer später dargestellten erfindungsgemäßen Wundauflage. Im linken oberen Teil der Figur 1 ist eine weibliche Brust 2 angedeutet. Die Wundauflage soll hierbei nach ihrer Fertigstellung beispielsweise eine durch Bestrahlung der Brust erzeugte Verbrennung (nicht dargestellt) abdecken. Zu diesem Zweck muss die Wundauflage so ausgestaltet sein, dass sie der Kontur der jeweiligen Brustpartie folgen kann. In Figur 1 ist die Wundauflage als Vorprodukt 1 dargestellt. Das Vorprodukt 1 ist in diesem Beispiel in Form einer Maschentechnik aufgebaut.

Zum Einsatz gelangen hierbei nicht oder nur geringfügig verstreckte biokompatible Polyesterfäden 3, wobei alternativ auch Polyamid-, oder Polypropylenfäden bzw. Seidenfäden eingesetzt werden können. Alternativ sind auch Wundauflagen einsetzbar, die Vorprodukte in Form einer Web- oder Vliesware aufweisen.

Ebenfalls dargestellt ist in vergrößerter Form ein Silikonfaden 4, der beispielsweise mit einer Polyesterummantelung 5 versehen ist. Alternativ zum Silikon kann auch ein Polyurethanfaden als Kernfaden verwendet werden.

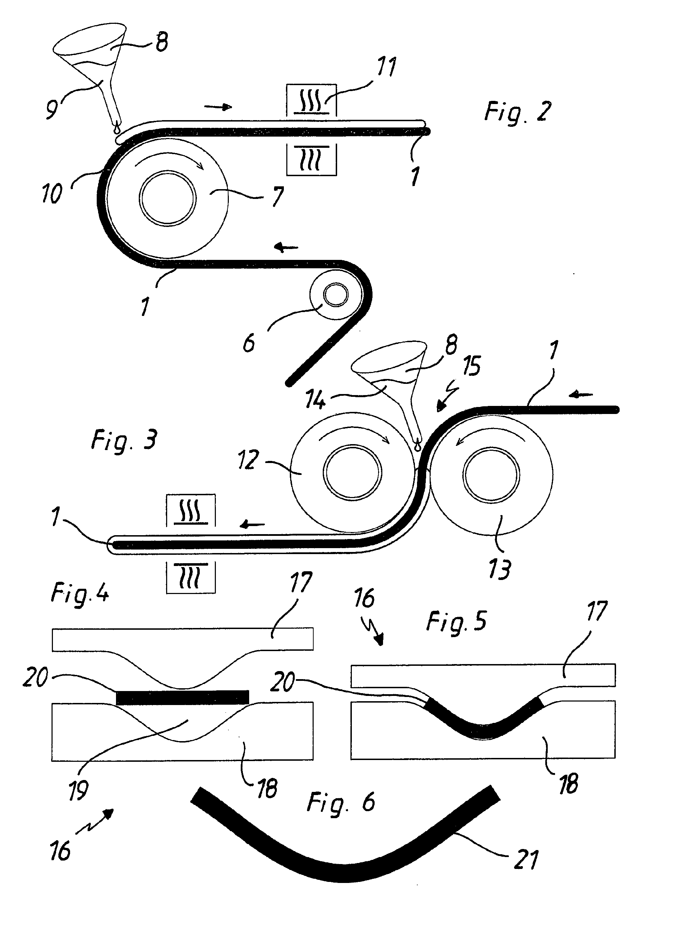

Die Figuren 2 und 3 zeigen unterschiedliche Beschichtungsverfahren für das in Figur 1 dargestellte Vorprodukt 1.

In Figur 2 wird das Vorprodukt 1 um mehrere Umlenkrollen 6, 7 geführt. Im Bereich der größeren Umlenkrolle 7 ist ein Aufgabetrichter 8 vorgesehen, der eine zuvor gemischte Masse, bspw. ein Zweikomponentensilikon 9, beinhaltet. Das Zweikomponentensilikon 9 wird auf die der Umlenkrolle 7 abgewandte Oberfläche 10 des Vorprodukts 1 aufgegeben, wobei unterschiedliche Schichtdicken erzeugt werden können. Im Zuge seines weiteren Transports wird das Vorprodukt 1 durch eine Heizeinrichtung 11 geführt. Hier kann entweder bereits eine vollständige Vernetzung des Zweikomponentensilikons oder aber lediglich eine teilweise Vernetzung vorgenommen werden.

Figur 3 zeigt eine Alternative zu Figur 2. Das hier dargestellte Vorprodukt 1 wird zwischen zwei beabstandeten Walzen 12, 13 umgelenkt. Auch hier ist ein Aufgabetrichter 8 vorgesehen, der in diesem Beispiel eine Masse 14 auf Basis von Vaseline oder Peru-Balsam in den Spalt 15 zwischen den Walzen 12, 13 einbringt. Alternativ und in Übereinstimmung mit Figur 1 kann das Vorprodukt ebenfalls mit einem Zweikomponentensilikon beschichtet werden. Abweichend zu Figur 2 wird das Vorprodukt 1 zwischen den Walzen 12, 13 beidseitig beschichtet. Durch Anpresskraft, Walzenabstand sowie Durchlaufgeschwindigkeit kann die Auftragsmenge des Beschichtungsmittels auf das Vorprodukt 1 variabel eingestellt werden. Das Vorprodukt 1 wird in Analogie zu Figur 2 durch eine Heizeinrichtung 11 geführt.

Die Figuren 4 und 5 zeigen als Prinzipskizzen eine Form 16, beinhaltend mehrere Formteile 17, 18, die zwischen sich eine Art Matrize 19 (Verformungsbereich) bilden. Vom Vorprodukt 1 gemäß Figur 2 oder 3 wurden entsprechende Stücke 20 abgetrennt und zwischen den Formteilen 17, 18 eingebracht. Figur 4 zeigt den geöffneten Zustand der Form 19, während Figur 5 den geschlossenen Zustand darstellt, in welcher das Teil 20 verformt wird. Die Form 19 kann bedarfsweise temperiert werden, um auf diese Art und Weise ein bei der Beschichtung nur teilweise vernetztes Vorprodukt dann vollständig zu vernetzen.

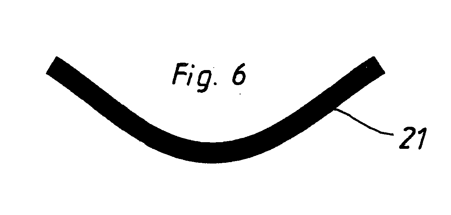

Figur 6 zeigt die verformte Wundauflage 21, die nun für die in Figur 1 dargestellte Brust 2 eingesetzt werden kann.

### Bezugszeichenliste

- 1: Vorprodukt
- 2: Brust
- 3: Polyesterfaden
- 4: Silikonfaden
- 5: Polyesterummantelung
- 6: Umlenkrolle
- 7: Umlenkrolle
- 8: Aufgabetrichter
- 9: Zweikomponentensilikon
- 10: Oberfläche
- 11: Heizeinrichtung
- 12: Walze
- 13: Walze
- 14: Peru-Balsam, Vaseline
- 15: Spalt
- 16: Form
- 17: Formteil
- 18: Formteil
- 19: Matrize
- 20: abgelängtes Vorprodukt
- 21: Wundauflage

## Patentansprüche

1. Verfahren zur Herstellung elastisch verformter Wundauflagen (21), indem biokompatible dehnbare Fäden oder Fasern (3, 4, 5) dergestalt miteinander in Wirkverbindung gebracht werden, dass ein etwa flächiges Vorprodukt (1) entsteht, dieses Vorprodukt (1) in Abhängigkeit der zum Einsatz gelangenden Fäden oder Fasern (3 - 5) bedarfsweise mit einer Paste oder einer Emulsion (9, 14) zumindest einseitig beschichtet wird und das Vorprodukt (1) zur Erlangung der gewünschten geometrischen Kontur anschließend einem Verformungsprozess unterzogen wird.

2. Verfahren nach Anspruch 1, **dadurch gekennzeichnet, dass** insbesondere nicht oder nur geringfügig verstreckte Fäden oder Fasern (3 - 5) auf Basis von Polyester, Polyamid oder Polypropylen eingesetzt werden.

3. Verfahren nach Anspruch 1, **dadurch gekennzeichnet, dass** Fäden oder Fasern (4, 5) auf Basis von Silikon oder Polyurethan eingesetzt werden, wobei die Fäden oder Fasern mit einer Ummantelung (5) auf Basis von Polyester, Polyamid oder Polypropylen versehen werden.

4. Verfahren nach einem der Ansprüche 1 - 3, **dadurch gekennzeichnet, dass** die Fäden oder Fasern (3 - 5) dergestalt miteinander in Wirkverbindung gebracht werden, dass flächige Gewebe-, Gestricke-, Gewirke- oder Non-woven-Vorprodukte (1) gebildet werden.

5. Verfahren nach einem der Ansprüche 1 - 4, **dadurch gekennzeichnet, dass** die Vorprodukte (1) mit einem biokompatiblen Silikon (9) beschichtet werden.

6. Verfahren nach Anspruch 5, **dadurch gekennzeichnet, dass** zur Beschichtung ein Zweikomponentensilikon (9) eingesetzt wird.

7. Verfahren nach einem der Ansprüche 1 - 4, **dadurch gekennzeichnet, dass** die Vorprodukte (1) mit Vaseline oder Peru-Balsam (14) beschichtet werden.

8. Verfahren nach einem der Ansprüche 1 - 7, **dadurch gekennzeichnet, dass** das Beschichten der Vorprodukte (1) durch Rakeln, Sprühen oder Foulardieren erfolgt.

9. Verfahren nach einem der Ansprüche 1 - 8, **dadurch gekennzeichnet, dass** der Verformungsprozess abgelängter Vorprodukte (20) innerhalb einer mit einer Matrize (19) versehenen Form (16) erfolgt.

10. Verfahren nach Anspruch 9, **dadurch gekennzeichnet, dass** die Verformung der Vorprodukte (20) entweder durch Druck oder durch Druck- und Temperatureinwirkung herbeigeführt wird.

11. Verfahren nach einem der Ansprüche 1 - 8, **dadurch gekennzeichnet, dass** der Verformungsprozess der Vorprodukte (20) durch Tiefziehen erfolgt.

12. Wundauflage (21), hergestellt nach dem Verfahren gemäß einem der Ansprüche 1-11.

13. Wundauflage (21) nach Anspruch 12, einsetzbar zur Abdeckung von äußeren Verletzungen der Haut, insbesondere im Bereich der Brust (2), des Knies, der Ferse oder des Ellenbogens.
